# EUROPÄISCHE PATENTSCHRIFT

(11) **EP 3 505 536 B1**
(45) Veröffentlichungstag und Bekanntmachung des Hinweises auf die Patenterteilung: **07.08.2024**
(21) Anmeldenummer: 18207370.0
(22) Anmeldetag: 29.06.2012
(51) Int. Cl.: C07K 16/28, C07K 16/30, A61P 35/00

(54) **ANTIKÖRPER GEGEN DAS PROSTATA-SPEZIFISCHE STAMMZELLANTIGEN UND DESSEN VERWENDUNG**
ANTIBODIES DIRECTED AGAINST THE PROSTATE-SPECIFIC STEM CELL ANTIGEN AND ITS USE
ANTICORPS CONTRE L'ANTIGÈNE DES CELLULES SOUCHES PROSTATIQUES ET LEUR UTILISATION

(30) Priorität: 30.06.2011 DE 102011118022
(43) Veröffentlichungstag der Anmeldung: 03.07.2019
(62) Teilanmeldung aus: 12729996.4
(73) Patentinhaber: AvenCell Europe GmbH, 01307 Dresden (DE)
(72) Erfinder: Bachmann, Michael, 65779 Kelkheim (DE)
(74) Vertreter: Kailuweit & Uhlemann Patentanwälte Partnerschaft mbB

(56) Entgegenhaltungen:
- WO-A1-01/05427
- WO-A2-01/40309
- WO-A2-2009/032949
- ERIC J LEPIN ET AL: "An affinity matured minibody for PET imaging of prostate stem cell antigen (PSCA)-expressing tumors", EUROPEAN JOURNAL OF NUCLEAR MEDICINE AND MOLECULAR IMAGING, SPRINGER, BERLIN, DE, vol. 37, no. 8, 1 April 2010 (2010-04-01), pages 1529 - 1538, XP019841981, ISSN: 1619-7089
- AGNIESZKA MORGENROTH: "Herstellung chimärer Rrezeptoren zur tumorspezifischen Armierung polyklonaler, zytotoxischer T-Lymphozyten", DISSERTATION ZUR ERLANGUNG DES AKADEMISCHEN GRADES DOCTOR RERUM NATURALIUM, TU DRESDEN, 7 December 2005 (2005-12-07), XP055037578, Retrieved from the Internet <URL:http://www.qucosa.de/fileadmin/data/qucosa/documents/1412/1134590060614-4888.pdf> [retrieved on 20120907]
- A. FELDMANN ET AL: "Novel Humanized and Highly Efficient Bispecific Antibodies Mediate Killing of Prostate Stem Cell Antigen-Expressing Tumor Cells by CD8+ and CD4+ T Cells", THE JOURNAL OF IMMUNOLOGY, vol. 189, no. 6, 15 September 2012 (2012-09-15), pages 3249 - 3259, XP055037485, ISSN: 0022-1767, DOI: 10.4049/jimmunol.1200341
- ANJA FELDMANN ET AL: "Retargeting of T cells to prostate stem cell antigen expressing tumor cells: Comparison of different antibody formats", THE PROSTATE, vol. 71, no. 9, 15 June 2011 (2011-06-15), pages 998 - 1011, XP055008882, ISSN: 0270-4137, DOI: 10.1002/pros.21315
- SCHWARZER A ED - SCHWARZER ET AL: "Herstellung und Charakterisierung rekombinanter Antikörper für eine adjuvante Immuntherapie PSCA-positiver Tumore", 1 January 2006 (2006-01-01), pages 22 - 24,96, XP002662580, Retrieved from the Internet <URL:http://swbplus.bsz-bw.de/bsz276658973inh.pdf>

## Beschreibung

Die Erfindung betrifft rekombinante an Prostata-spezifisches Stammzellantigen (PSCA) bindende Antikörper und deren Verwendung in der Diagnostik und Therapie. Die Antikörper eignen sich zur Anwendung im Bereich der Medizin, Pharmazie und der biomedizinischen Forschung.

Das Prostatakarzinom ist eine der häufigsten krebsbedingten Todesursachen in Deutschland. Die Standardtherapie bei einem primären, organbegrenzten Prostatakarzinom ist derzeit die radikale Prostatektomie, d. h. die vollständige Entfernung von Prostata, Samenblasen und Lymphknoten. Eine Alternative dazu stellt die Strahlentherapie dar, die durch Spickung der Prostata mit radioaktivem Material (Brachytherapie) erfolgt. Die meisten Patienten mit primärem, lokalem Prostatakarzinom können erfolgreich durch radikale Prostatektomie und Strahlentherapie behandelt werden. Eine Rezidivbildung tritt bei etwa 20 - 40 % der Betroffenen auf [Roehl 2004].

Gegenwärtig werden verschiedene antikörperbasierte Therapieverfahren entwickelt. Dabei ist es von entscheidender Bedeutung, Zielstrukturen auf den Krebszellen zu identifizieren, die als Angriffsstellen für die antikörperbasierten Therapeutika dienen können. Eine geeignete Zielstruktur als Angriffsstelle für die Behandlung von Prostatakrebs sind Oberflächenproteine, die hauptsächlich im Prostatagewebe vorliegen und die eine Überexpression auf malignen im Vergleich zu gesunden Zellen zeigen.

Das Prostata-spezifische Stammzellantigen (PSCA) ist ein derartiges Oberflächenmolekül, das spezifisch auf Prostatazellen vorliegt und das in Prostatakarzinomzellen im Vergleich zum gesunden Gewebe vermehrt exprimiert wird (tumorassoziiertes Antigen). Humanes PSCA umfasst 123 Aminosäuren und weist eine N-terminale Signalsequenz, eine C-terminale Glykosylphosphatidylinositol (GPI)-Verankerungssequenz und mehrere N-Glykosylierungsstellen auf. PSCA wird zur Thy-1/Ly-6 Familie der GPI-verankerten Zelloberflächenmoleküle gezählt, da es u.a. die für diese Familie charakteristischen konservierten Cystein-Reste aufweist [Reiter 1998]. Aufgrund der erhöhten Expression in Prostatatumoren ist PSCA ein geeignetes Zielmolekül für die Therapie von Prostatakrebs und es wurden bislang verschiedene Therapiestrategien, die auf PSCA als Target abzielen, entwickelt. Ein Nachweis der Wirksamkeit von PSCA als therapeutischem Target bei der Krebstherapie am Menschen wurde u.a. durch die Vakzinierung mit dendritischen Zellen, die zuvor mit einem PSCA-Peptid beladen wurden, erbracht. Klinische Studien (Phase 1 und 2) mit 12 Hormon- und Chemotherapie-refraktären Prostatakarzinom-Patienten zeigten, dass eine Vakzinierung mit PSCA-Peptid-beladenen Dendritischen Zellen bei fünf der Patienten eine T-Zell-Antwort gegen PSCA verursachte, die im Mittel mit einer verlängerten Überlebenszeit korrelierte. Bei einem Patienten konnte sogar eine Reduktion der Tumormasse beobachtet werden [Thomas-Kaskel 2006].

WO 2009/032949 A2 offenbart monoklonale anti-PSCA-Antikörper (1G8), die zum Targeting von Tumoren und zu deren Detektion eingesetzt werden. Die CDR-Regionen der Antikörper haben die folgenden Aminosäuresequenzen:

| | variable Region der leichten Kette (1G8) | | variable Region der schweren Kette (1G8) | |
|---|---|---|---|---|
| CDR1 | SASSSVRFIHW | SEQ ID No. 69 | DYYIHW | SEQ ID No. 72 |
| CDR2 | DTSKLAS | SEQ ID No. 70 | WIDPENGDTEFVPKFQG | SEQ ID No. 73 |
| CDR3 | QQWSSSPFT | SEQ ID No. 71 | TGGF | SEQ ID No. 74 |

Aufgrund der zytotoxischen Eigenschaften des 1G8 Antikörpers ist dieser für Targetingstrategien, die auf der Rekrutierung von Effektorzellen beruhen, ungeeignet (Gu 2005).

Die in WO 2009/032949 A2 offenbarten Antikörper werden diagnostisch bevorzugt in Form von funktionellen murinen und humanisierten monoklonalen Antikörpern sowie mit Radionukliden markierten PSCA-spezifischen Minibodies und Diabodies eingesetzt.

1G8 und weitere anti-PSCA-Antikörper sind in WO 01/40309 A2, WO 01/05427 A1, Lepin et al. 2010 und Morgenroth 2005 offenbart.

[Feldmann 2011] entwickelten bispezifische rekombinante Antikörper mit einem PSCA-bindenden Paratop und einem CD3-bindenden Paratop. Das PSCA-bindende Paratop der bispezifischen Antikörper ist vom PSCA-Antikörper 7F5 abgeleitet [Morgenroth 2007] und umfasst CDR-Regionen mit den folgenden Aminosäuresequenzen:

| | variable Region der leichten Kette (7F5) | | variable Region der schweren Kette (7F5) | |
|---|---|---|---|---|
| CDR1 | RTSQDISNYLN | SEQ ID No. 27 | SYTMS | SEQ ID No. 30 |
| CDR2 | YTLKLNS | SEQ ID No. 28 | YIHNGGGHTYYPDTIKG | SEQ ID No. 31 |
| CDR3 | QQSKTLPWT | SEQ ID No. 29 | RMYYGNSHWYFDV | SEQ ID No. 32 |

Die in [Feldmann 2011] offenbarten bispezifischen Antikörper führten *in vitro* erfolgreich zur spezifischen T-Zell-vermittelte Lyse PSCA-positiver Tumorzellen. Für eine spezifische Lyse von etwa 40 % der eingesetzten PSCA-positiven Tumorzellen ist bei einem Verhältnis von Effektorzellen zu PSCA-positiven Tumorzellen von 20:1 mindestens 5 ng der in [Feldmann 2011] offenbarten bispezifischen Antikörper einzusetzen. In vitro konnte mit dem in [Feldmann 2011] offenbarten Antikörper eine spezifische Lyse von maximal etwa 60 % der eingesetzten PSCA-positiven Tumorzellen erreicht werden (in Gegenwart von 50 - 100 ng des Antikörpers). Eine weitere Erhöhung der Effektivität konnte nicht nachgewiesen werden. Auch in Gegenwart von 1000 ng des bispezifischen Antikörpers konnte kein höherer Anteil PSCA-positiver Tumorzellen lysiert werden.

Die bekannten *in vitro* und *in vivo* Daten zeigen, dass PSCA ein großes Potenzial als Zielantigen für die Immuntherapie von Prostatakarzinomen besitzt und als diagnostisches Target geeignet ist.

Aufgabe der Erfindung ist es, verbesserte Antikörper gegen PSCA bereitzustellen, die insbesondere in Form von bispezifischen rekombinanten Antikörpern in geringer Konzentration therapeutisch wirksam sind und PSCA-positive Tumorzellen effektiv lysieren können.

Die Aufgabe wird erfindungsgemäß gelöst durch einen Antikörper (hierin auch als anti-PSCA Antikörper bezeichnet) umfassend mindestens zwei unterschiedliche Bindungseinheiten,
i. mindestens eine der Bindungseinheiten an Prostata-spezifisches Stammzellantigen (PSCA) bindet, wobei
   - eine variable Region der leichten Kette die Aminosäuresequenzen: SEQ ID No. 1, SEQ ID No. 2, SEQ ID No. 3 umfasst und
   - eine variable Region der schweren Kette die Aminosäuresequenzen: SEQ ID No. 4, SEQ ID No. 5 und SEQ ID No. 6 umfasst;
ii. mindestens eine weitere der Bindungseinheiten gegen CD3 gerichtet ist.

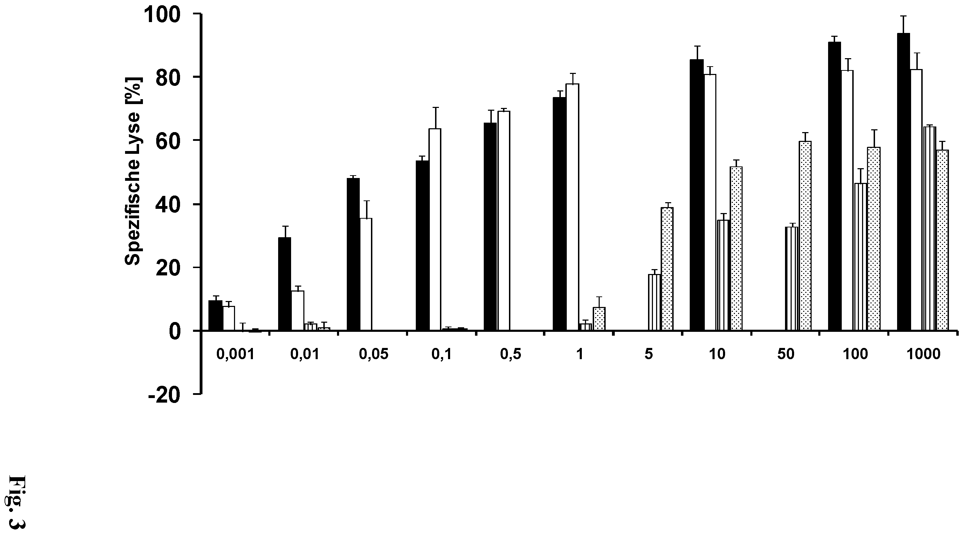

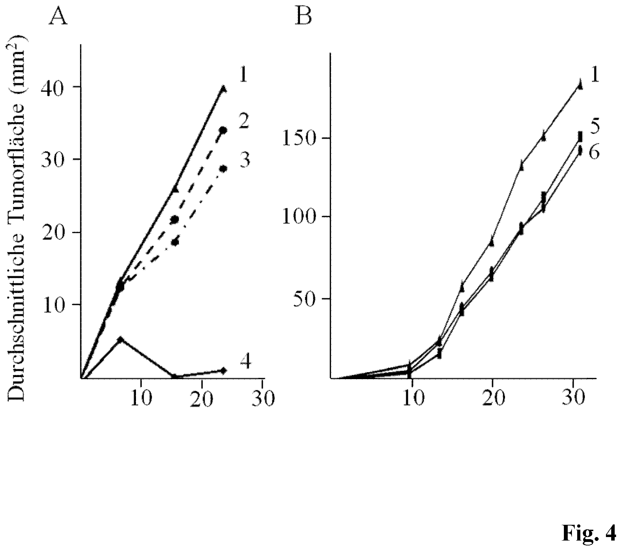

Der erfindungsgemäße Antikörper, der durch die vorgenannten Sequenzen gekennzeichnet ist, wird hierin auch vereinfacht als MB1 bezeichnet. Die Erfinder stellten im Rahmen umfangreicher Untersuchungen überraschend fest, dass der erfindungsgemäße Antikörper in Form von rekombinant hergestellten bispezifischen Antikörpern die spezifische Lyse PSCA-positiver Tumorzellen auch in sehr geringen Mengen vermitteln kann. Ferner wurde überraschend festgestellt, dass die durch die neuen Antikörper vermittelte spezifische Lyse von PSCA-positiven Tumorzellen effektiver ist. So konnten in *in vitro* Untersuchungen mit bispezifischen Antikörpern, die einen erfindungsgemäßen anti-PSCA Antikörper enthalten über 90 % der eingesetzten PSCA-positiven Tumorzellen lysiert werden. Es kann mit dem neuen anti-PSCA Antikörper somit die eingesetzte Antikörpermenge deutlich reduziert werden. Gleichzeitig wird die therapeutische Wirksamkeit erhöht, da mit dem erfindungsgemäßen Antikörper PSCA-positive Tumorzellen effektiver lysiert werden können. Aufgrund der geringen Konzentration, in der mit dem erfindungsgemäßen Antikörper eine Wirksamkeit erzielt werden kann, ist mit dem erfindungsgemäßen Antikörper auch eine verbesserte Abtötung von metastasierenden PSCA-positiven Zellen möglich. Vergleichsuntersuchungen mit dem aus dem Stand der Technik bekannten anti-PSCA 7F5 in einem vergleichbaren bispezifischen Konstrukt bestätigen deutlich die Überlegenheit des erfindungsgemäßen Antikörpers sowohl in *in vitro* (Fig. 3) als auch *in vivo* Studien (Fig. 4).

Bevorzugte erfindungsgemäße anti-PSCA Antikörper enthalten die wie vorstehend definierten Sequenzen, wobei die gegen PSCA gerichtete Bindungseinheit eine Aminosäuresequenz der variablen Regionen der leichten und schweren Kette mindestens 80 %, bevorzugt mindestens 90 %, besonders bevorzugt mindestens 95%, Aminosäure-Sequenzidentität zu folgenden Aminosäuresequenz aufweist:
- variable Region der leichten Kette SEQ ID No. 24, variable Region der schweren Kette SEQ ID No. 26 oder
- variable Region der leichten Kette SEQ ID No. 20, variable Region der schweren Kette SEQ ID No. 22.

Bevorzugt sind davon anti-PSCA Antikörper mit den oben definierten Sequenzen, dessen variable Regionen eine humanisierte Struktur aufweisen. Besonders bevorzugt sind anti-PSCA Antikörper, deren variable Region der leichten Kette die Aminosäuresequenz gemäß SEQ ID No. 24 und deren variable Region der schweren Kette die Aminosäuresequenz gemäß SEQ ID No. 26 enthält.

Der Begriff "Antikörper" im erfindungsgemäßen Sinn umfasst alle Antikörper oder Antikörperfragmente, die in der Lage sind, spezifisch an ein Antigen zu binden. Bei rekombinanten Antikörpern handelt es sich dabei um Antikörper, die mit Hilfe von gentechnisch veränderten Organismen hergestellt werden. Der Begriff Antikörper umfasst sowohl die kompletten monoklonalen Antikörper als auch deren epitopbindende Fragmente. Hierbei umfassen die epitopbindenden Fragmente (hier auch als Antikörperfragmente bezeichnet) diejenigen Teile des Antikörpers, die in der Lage sind, an das Antigen zu binden. Antikörperfragmente im Sinne der Erfindung beinhalten Fab, Fab', F(ab')₂, Fd, Einzelketten (single-chain) variable Fragmente (scFv), Einzelketten-Antikörper, disulfidverlinkte variable Fragmente (sdFv) und Fragmente die entweder eine variable Region der leichten Kette (V_{L}) oder eine variable Region der schweren Kette (V_{H}) enthalten. Antikörperfragmente enthalten die variablen Regionen entweder allein oder in Kombination mit weiteren Regionen, die ausgewählt sind aus der Hinge-Region, und dem ersten, zweiten und dritten Bereich der konstanten Region (C_{H}1, C_{H}2, C_{H}3).

Weiterhin umfasst der Begriff Antikörper rekombinant hergestellte Antikörper, wie Diabodies, Triabodies und Tetrabodies. Ebenfalls vom Begriff Antikörper umfasst sind chimäre Antikörper, bei denen unterschiedliche Teile des Antikörpers aus verschiedenen Spezies stammen, wie beispielsweise Antikörper mit einer murinen variablen Region, welche mit einer humanen konstanten Region kombiniert ist. Humanisierte Antikörper sind hierin ebenfalls durch den Begriff Antikörper umfasst. Das Ziel der Humanisierung von Antikörpern liegt in der Reduktion der Immunogenität eines xenogenen Antikörpers, wie beispielsweise muriner Antikörper, zur Verwendung im humanen System, wobei die volle Bindungsaffinität und die Antigenspezifität erhalten bleiben. Humanisierte Antikörper können auf verschiedenen bekannten Wegen hergestellt werden, wie beispielsweise durch Resurfacing und CDR-Grafting. Beim Resurfacing werden durch eine Kombination aus Molecular Modelling, statistischen Analysen und Mutagenese alle nicht-CDR-Regionen auf der Oberfläche des Antikörpers verändert, so dass diese der Oberfläche von Antikörpern des Zielorganismus ähneln. Beim CDR-Grafting werden die CDR-Regionen des zu humanisierenden Antikörpers in humane variable Regionen eingebracht.

Antikörperfragmente sind gegebenenfalls untereinander über einen Linker verknüpft. Der Linker umfasst eine kurze (bevorzugt 10 bis 50 Aminosäurereste lange) Peptidsequenz, die so ausgewählt wird, dass das Antikörperfragment eine derartige dreidimensionale Faltung der V_{L} und V_{H} besitzt, dass es die Antigenspezifität des kompletten Antikörpers aufweist. Bevorzugt sind Glycin-Serin-Linker oder Linkerpeptide mit einer Aminosäuresequenz gemäß SEQ ID No. 75 oder SEQ ID No. 76.

Die Bezeichnung "variable Region" bedeutet hierin die Teile der schweren und leichten Ketten der Antikörper, die sich in ihrer Sequenz zwischen Antikörpern unterscheiden und die Spezifität des Antikörpers und die Bindung an sein Antigen bestimmen. Die Variabilität ist hierbei nicht gleichmäßig in der variablen Region verteilt, sondern ist üblicherweise innerhalb dreier definierter Segmente der variablen Region konzentriert, den komplementaritätsbestimmenden Regionen (CDRs, auch als hypervariable Regionen bezeichnet), die in den variablen Regionen sowohl der leichten als auch der schweren Kette enthalten sind. Die Antigenbindungsstelle eines Antikörpers, das sogenannte Paratop, ist durch die hypervariablen Regionen (CDR) der leichten und schweren Ketten des Antikörpers charakterisiert.

Zur Beschreibung von Antikörpern wird hierin auch die vereinfachte Bezeichnung anti-"Antigen" Antikörper verwendet, um darzustellen, dass es sich dabei um einen Antikörper handelt, der spezifisch an das in der Bezeichnung angegebene Antigen bindet. So ist beispielsweise unter einem "anti-PSCA Antikörper" im Sinn der Erfindung ein Antikörper zu verstehen, der spezifisch an das Antigen PSCA bindet. Unter einer spezifischen Bindung eines Antikörpers an ein bestimmtes Antigen wird hierin verstanden, dass ein Antikörper mit einer hohen Affinität an das bestimmte Antigen bindet und mit einer deutlich geringeren Affinität und vorzugsweise nicht an andere Antigene bindet.

Bevorzugte Antikörper liegen in Form eines scFv-Fragments oder eines F(ab')₂-Fragments vor. Weiter bevorzugte Antikörper sind bispezifische Antikörper, die rekombinant hergestellt werden.

Bevorzugt sind erfindungsgemäße anti-PSCA Antikörper (naive oder rekombinante Antikörper) mit einer Effektorgruppe konjugiert. Konjugation bedeutet hierbei die Ankopplung eines Stoffs, also der Effektorgruppe, an den Antikörper. Die Verbindung des Antikörpers zur Effektorgruppe wird bevorzugt durch rekombinante Expression in Form eines Fusionsproteins oder durch *in vitro* Methoden hergestellt, wobei die Effektorgruppe bevorzugt über chemische Linkergruppen an den Antikörper gebunden wird (beispielsweise über Thioetherbindungen oder Disulfidbindungen). Sie können an den Antikörper auch durch ein intermediäres Trägermolekül, beispielsweise Serumalbumin, gebunden sein. Gegebenenfalls enthält ein erfindungsgemäßer Antikörper mehrere Effektorgruppen. Effektorgruppen sind dabei vorzugsweise ausgewählt aus Wirkstoffen, Peptiden (mit einer Länge von 10 bis 100 Aminosäuren), Proteinen (mit einer Länge von mehr als 100 Aminosäuren), costimulatorischen Molekülen, Farbstoffen oder Kontrastmitteln.

Bevorzugte erfindungsgemäße anti-PSCA Antikörper sind mit Wirkstoffen, also mit pharmazeutisch wirksamen Stoffen, konjugiert. Bevorzugte Wirkstoffe umfassen Toxine, vorzugsweise Zytostatika, davon bevorzugt ausgewählt aus Maytansinoiden und Maytansinoid-Analoga, Taxoiden, CC-1065 und CC-1065 Analoga, Dolastatin und Dolastatin-Analoga, Methotrexat, Daunorubicin, Doxorubicin, Vincristin, Vinblastin, Melphalan, Mitomycin C, Chlorambucil und Calicheamicin.

Weiter bevorzugte erfindungsgemäße anti-PSCA Antikörper sind mit Kontrastmitteln konjugiert. Bevorzugte Kontrastmittel sind Radionuklide, davon bevorzugt die radioaktiven Isotope von Technetium, Rhenium, Yttrium, Kupfer, Gallium, Indium, Bismuth und Platin, insbesondere ^{99m}Tc, ⁹⁰Y, ¹⁸⁶Re, ¹⁸⁸Re, ⁶⁸Ga und ¹¹¹In.

Weiter bevorzugte erfindungsgemäße anti-PSCA Antikörper sind mit einem Protein, bevorzugt einem Enzym, vorzugsweise einem für das ADEPT-System geeigneten Enzym, einem costimulatorischen Molekül, vorzugsweise Toll-like Rezeptor Ligand, davon bevorzugt CpG oder mit einer Nukleinsäure konjugiert.

Weiter bevorzugte erfindungsgemäße anti-PSCA Antikörper sind mit einem Farbstoff, vorzugsweise einem Fluoreszenzfarbstoff konjugiert.

Weiter bevorzugte erfindungsgemäße Antikörper sind mit einem Peptid konjugiert, das eine Bindungsregion enthält, an die ein für das Peptid spezifischer Antikörper spezifisch bindet. Bevorzugt umfasst das Peptid eine 10 bis 50 Aminosäuren lange Peptidsequenz einer alpha-helikalen Region des humanen La-Proteins (die Aminosäuresequenz des humanen La Proteins entspricht SEQ ID No. 77). Besonders bevorzugt sind Peptide mit einer Aminosäure-Sequenzidentität von mindestens 90%, bevorzugt mindestens 95 %, besonders bevorzugt mindestens 99%, zu einer der Aminosäuresequenzen gemäß SEQ ID No. 75 oder SEQ ID No. 76.

Besonders bevorzugt werden erfindungsgemäße anti-PSCA Antikörper rekombinant hergestellt.

Unter einer "Bindungseinheit" im Sinne der Erfindung wird jegliche molekulare Struktur bezeichnet, die definierte Substanzen oder Strukturen spezifisch bindet. Abhängig von der Substanz oder Struktur, die gebunden wird, weist die Bindungseinheit dabei verschiedene Strukturen auf. Von der Bezeichnung "Bindungseinheit" im Sinne der Erfindung sind daher sowohl Antikörper umfasst (in diesem Fall enthält die Bindungseinheit zumindest das funktionale Paratop des Antikörpers) als auch andere Moleküle, vorzugsweise Proteine, die eine spezifische Bindung mit einem Antigen eingehen. Derartige Moleküle sind vorzugsweise Liganden, die spezifisch an eine Oberflächenstruktur (beispielsweise einen Oberflächenrezeptor) auf einer Zelle, insbesondere einer Effektorzelle, binden.

Erfindungsgemäß bindet die weitere Bindungseinheit, die in erfindungsgemäßen Antikörpern enthalten ist, spezifisch an eine Effektorzelle. Die Definition von Effektorzellen im Sinne der Erfindung umfasst alle die Zellen des angeborenen und adaptiven Immunsystems, die Immunreaktionen vermitteln oder aktiv daran beteiligt sind. Bevorzugt ist die Effektorzelle ausgewählt aus T-Lymphozyten, NK-Zellen, Monozyten, Makrophagen, Dendritischen Zellen und Granulozyten.

Erfindungsgemäß enthält der rekombinante Antikörper mindestens zwei unterschiedliche Antikörper (mindestens einen erfindungsgemäßen anti-PSCA Antikörper und mindestens einen weiteren Antikörper, der gegen CD3 gerichtet ist), bevorzugt in Form eines Diabody, Triabody oder Tetrabody. Bevorzugt davon sind bispezifische Antiköper aus Einzelketten-Antikörpern (single chain bispecific diabody, scBsDb) oder bispezifische Tandemantikörper aus Einzelketten-Antikörpern (single chain bispecific tandem antibody, scBsTaFv). Ganz besonders bevorzugt liegt ein erfindungsgemäßer rekombinanter Antikörper in Form eines scBsTaFv vor.

Erfindungsgemäß ist der Antikörper gegen die folgende Oberflächenstruktur auf Effektorzellen gerichtet: CD3. Bevorzugt sind Antikörper, die gegen CD3 gerichtet sind (anti CD3-Antikörper), wobei der anti-CD3 Antikörper die folgenden Aminosäuresequenzen enthält:
- eine variable Region der schweren Kette: SEQ ID No. 66, SEQ ID No. 67, SEQ ID No. 68 und
- eine variable Region der leichten Kette: SEQ ID No. 58, SEQ ID No. 59, SEQ ID No. 60.

Weiter bevorzugt sind rekombinante anti-PSCA Antikörper, die mindestens zwei unterschiedliche Bindungseinheiten enthalten, wobei mindestens eine weitere der Bindungseinheiten ein Ligand (bevorzugt ein Protein oder ein Glykan) ist, der an eine Oberflächenstruktur auf einer Effektorzelle spezifisch bindet. Bevorzugte Liganden beeinflussen die Aktivität der Effektorzellen durch deren Bindung an die (Effektor-)Zelloberfläche. Der Ligand ist dabei so ausgewählt, dass er spezifisch an Oberflächenstrukturen von Effektorzellen bindet und durch die Bindung Signalkaskaden zur Aktivierung der Effektorzellen auslöst. Bevorzugt ist als Ligand eine Proteinstruktur oder ein Glykan, welcher spezifisch an einen Rezeptor bindet, der spezifisch auf der Oberfläche von Effektorzellen exprimiert wird, wobei der Ligand durch seine Bindung an den Rezeptor eine Aktivierung der Effektorzelle bewirkt. Besonders bevorzugt sind die Proteinstrukturen ausgewählt aus ULB-Ps (z. B. ULB-P2), MICA, MICB und Zytokinen (wie z.B. IL2 und IL15).

Erfindungsgemäße Antikörper, insbesondere erfindungsgemäße rekombinante Antikörper, eignen sich zur spezifischen Bindung an PSCA-positive Zellen *in vivo* und *in vitro.* Daher umfasst die Erfindung auch die Verwendung der erfindungsgemäßen anti-PSCA Antikörper als Arzneimittel, insbesondere zur Behandlung von Tumorerkrankungen, bevorzugt Prostatakrebs, oder als Diagnostikum.

Die Erfindung umfasst ferner die erfindungsgemäßen Antikörper zur Verwendung in der Therapie von Tumorerkrankungen, insbesondere Prostatakrebs. Ebenfalls von der Erfindung umfasst ist die Verwendung der erfindungsgemäßen Antikörper zur Herstellung eines Arzneimittels zur Behandlung von Tumorerkrankungen, insbesondere Prostatakrebs.

Eine bevorzugte therapeutische Anwendung der erfindungsgemäßen (bevorzugt rekombinanten) Antikörper ist die Behandlung von Tumorerkrankungen, vorzugsweise Prostatakrebs. In der therapeutischen Anwendung werden die erfindungsgemäßen Antikörper bevorzugt zum Targeting von therapeutisch wirksamen Substanzen oder Effektorzellen zum Tumorgewebe eingesetzt. Zum Targeting von therapeutisch wirksamen Substanzen zum Tumorgewebe kommen bevorzugt erfindungsgemäße rekombinante Antikörper zum Einsatz, die mit einem Wirkstoff konjugiert sind. Zum Targeting von Effektorzellen zum Tumorgewebe werden erfindungsgemäße rekombinante Antikörper eingesetzt, die mindestens zwei unterschiedliche Bindungseinheiten enthalten, wobei mindestens eine der Bindungseinheiten ein erfindungsgemäßer anti-PSCA Antikörper ist und eine weitere der Bindungseinheiten spezifisch an CD3 auf T Zellen, bindet. Bevorzugt werden dafür die obenstehend genannten Antikörper eingesetzt.

Von der Erfindung ist auch eine pharmazeutische Zusammensetzung umfasst, die einen erfindungsgemäßen Antikörper in Assoziation mit einem pharmazeutisch annehmbaren Verdünnungsmittel oder Träger enthält. Bevorzugt liegt die erfindungsgemäße pharmazeutische Zusammensetzung in einer für die intravenöse Verabreichung geeigneten Form vor.

Vorzugsweise liegen die Antikörper in der erfindungsgemäßen pharmazeutischen Zusammensetzung in rekombinanter Form als chimäre oder besonders bevorzugt humanisierte Antikörper vor, die eine verringerte Immunogenität aufweisen.

Die erfindungsgemäßen pharmazeutischen Zusammensetzungen umfassen verschiedene Dosierungsformen und sind bevorzugt für die parenterale, besonders bevorzugt für die intravenöse Verabreichung geeignet. Vorzugsweise liegt die parenterale pharmazeutische Zusammensetzung in einer Darreichungsform vor, die zur Injektion geeignet ist. Besonders bevorzugte pharmazeutische Zusammensetzungen sind daher Lösungen, Emulsionen oder Suspensionen des Antikörpers in einem pharmazeutisch annehmbaren Verdünnungsmittel oder Träger.

Pharmazeutisch annehmbare Träger sind vorzugsweise sterile Flüssigkeiten, insbesondere Wasser, gepuffertes Wasser, 0,4% Salzlösung, 0,3% Glycin und dergleichen. Die pharmazeutischen Zusammensetzungen werden durch herkömmliche, gut bekannte Techniken sterilisiert. Die Zusammensetzungen enthalten bevorzugt pharmazeutisch akzeptable Hilfssubstanzen, wie etwa diejenigen, die erforderlich sind um näherungsweise physiologische Bedingungen zu ergeben und/oder die Stabilität der in der Zusammensetzung enthaltenen Antikörper zu erhöhen, wie etwa Mittel zur Einstellung des pH-Werts und Puffermittel, Mittel zur Einstellung der Toxizität und dergleichen, vorzugsweise ausgewählt aus Natriumacetat, Natriumchlorid, Kaliumchlorid, Calciumchlorid und Natriumlactat.

Bevorzugt ist die pharmazeutische Zusammensetzung eine injizierbare gepufferte Lösung, die zwischen 0,1 bis 500 mg/ml Antikörper, besonders bevorzugt zwischen 0,1 bis 250 mg/ml Antikörper, insbesondere zusammen mit 1 bis 500 mMol/l Puffer, enthält. Die injizierbare Lösung liegt bevorzugt sowohl in einer flüssigen oder in einer lyophilisierten Dosierungsform vor. Der Puffer ist bevorzugt ausgewählt aus Histidin, Natriumsuccinat, Natriumcitrat, Natriumphosphat und Kaliumphosphat.

Eine bevorzugte diagnostische Anwendung ist die *in vivo* Diagnostik, wobei ein mit Kontrastmittel konjugierter erfindungsgemäßer anti-PSCA Antikörper zum gezielten Transport von Kontrastmitteln zum Tumorgewebe eingesetzt wird. Eine weiter bevorzugte diagnostische Anwendung der erfindungsgemäßen anti-PSCA Antikörper ist die *in vitro* Diagnostik, wobei bevorzugt ein mit einem Farbstoff konjugierter erfindungsgemäßer anti-PSCA Antikörper zum Nachweis PSCA-positiver Zellen in einer Probe, insbesondere in einer Gewebeprobe, eingesetzt wird.

Von der Erfindung ist auch eine diagnostische Zusammensetzung umfasst, die einen erfindungsgemäßen anti-PSCA Antikörper enthält. Der anti-PSCA Antikörper liegt darin vorzugsweise in einer Pufferlösung vor, vorzugsweise in gepufferter Salzlösung.

Die Erfindung umfasst ebenfalls eine Nukleinsäure, dessen Nukleotidsequenz für einen erfindungsgemäßen anti-PSCA Antikörper kodiert. Bevorzugt enthalten die für die variablen Regionen der leichten und schweren Kette kodierenden Abschnitte folgende Nukleotidsequenzen:
- variable Region der leichten Kette: SEQ ID No. 7, SEQ ID No. 9, SEQ ID No. 11 und variable Region der schweren Kette: SEQ ID No. 13, SEQ ID No. 15, SEQ ID No. 17 oder
- variable Region der leichten Kette: SEQ ID No. 8, SEQ ID No. 10, SEQ ID No. 12 und variable Region der schweren Kette: SEQ ID No. 14, SEQ ID No. 16, SEQ ID No. 18.

Der Begriff "Nukleinsäuren" im Sinne der Erfindung umfasst neben Desoxyribonukleinsäuren (DNA) und Ribonukleinsäuren (RNA) auch alle anderen linearen Polymere, in denen die Basen Adenin (A), Cytosin (C), Guanin (G) und Thymin (T) oder Uracil (U) in entsprechender Abfolge angeordnet sind (Nukleinsäuresequenz). Die Erfindung umfasst dabei auch die korrespondierenden RNA-Sequenzen (in denen Thymin durch Uracil ersetzt ist), komplementäre Sequenzen und Sequenzen mit modifiziertem Nukleinsäurerückgrat oder 3' oder 5'-Terminus. Der Begriff "Nukleinsäuresequenzen mit verändertem Rückgrat" umfasst dabei alle anderen linearen Polymere in denen die Basen Adenin (A), Cytosin (C), Guanin (G) und Thymin (T) oder Uracil (U) in entsprechender Abfolge angeordnet sind, wie z. B. Sequenzen mit einem Phosphothioat-, Phosphoramidat- oder O-Methyl-derivatisierten Rückgrat, Peptid-Nukleinsäuren (PNA) und locked nucleic acids (LNA) oder gemischtem Rückgrat. Der Begriff "modifizierter 3' oder 5' Terminus" umfasst dabei sowohl Modifikationen, die der Stabilisierung dienen als auch das Anbinden von Markern. Beispiele für Marker sind Enzyme, Farbstoffe oder Fluoreszenzfarbstoffe, Radionukleotide, sowie Haptene, wie z. B. Digoxigenin oder Biotin.

Von der Erfindung ist auch ein Vektor (auch: "Expressionsvektor") umfasst, der eine erfindungsgemäße Nukleinsäure enthält. Im Sinne der Erfindung wird unter einem Expressionsvektor ein Plasmid, Virus oder anderer Träger verstanden, der eine erfindungsgemäße Nukleinsäuresequenz rekombinant durch Insertion oder Inkorporation enthält. Der Expressionsvektor enthält typischerweise einen Replikationsstartpunkt, einen Promoter, sowie spezifische Gensequenzen, die eine phänotypische Selektion von den Expressionsvektor enthaltenden Wirtszellen ermöglichen.

Die Erfindung umfasst ferner eine Wirtszelle oder einen nicht-menschlichen Wirtsorganismus enthaltend eine erfindungsgemäße Nukleotidsequenz oder einen erfindungsgemäßen Vektor. Die Nukleotidsequenz oder der Vektor sind dabei rekombinant in der Wirtszelle oder dem nicht-menschlichen Wirtsorganismus enthalten.

Eine Wirtszelle im Sinne der Erfindung ist eine natürlich vorkommende Zelle oder eine transformiert oder genetisch veränderte Zelllinie, welche mindestens einen erfindungsgemäßen Vektor enthält. Die Erfindung umfasst dabei transiente Transfektanten (z. B. durch mRNA-Injektion) oder Wirtszellen, in denen mindestens ein erfindungsgemäßer Expressionsvektor als Plasmid oder künstliches Chromosom enthalten ist, sowie Wirtszellen in denen ein erfindungsgemäßer Expressionsvektor stabil in das Genom des Wirtes integriert ist.

Die Wirtszelle ist bevorzugt ausgewählt aus Zellen von Prokaryonten und Eukaryonten. Humane embryonale Stammzellen, die unter Vernichtung von Embryonen gewonnen wurden, sind keine Wirtszellen im Sinne der Erfindung. Bevorzugte Prokaryontenzellen sind ausgewählt aus Zellen von *Escherichia coli* und *Bacillus subtilis.* Bevorzugte Eukaryontenzellen sind ausgewählt aus Hefezellen (vorzugsweise *Saccharomyces cerevisiae* oder *Pichia pastoris),* Insektenzellen, amphibischen Zellen und Säugetierzellen (vorzugsweise CHO, HeLa, HEK293).

Nicht-menschliche Wirtsorganismen enthalten einen erfindungsgemäßen Vektor, der stabil in das Genom des Wirtsorganismus oder einzelner Zellen des Wirtsorganismus integriert ist. Bevorzugte Wirtsorganismen sind Pflanzen, Invertebraten oder Vertebraten, insbesondere *Bovidae, Drosophila melanogaster, Caenorhabditis elegans, Xenopus laevis,* Medaka, Zebrafisch oder *Mus musculus,* oder Zellen oder Embryonen der genannten Organismen.

Die Erfindung gibt anti-PSCA Antikörper und dazugehörige Expressionssysteme an, mit denen humanes PSCA effektiver gebunden werden kann. Dadurch eignet sich der erfindungsgemäße anti-PSCA Antikörper besonders für den Einsatz in Therapiesystemen, bei denen die Abtötung von Tumorzellen durch Rekrutierung von Effektorzellen vermittelt werden kann. Aufgrund der höheren Affinität des erfindungsgemäßen Antikörpers zu PSCA ist für eine Bindung (beispielsweise in der therapeutischen Anwendung) nur noch eine wesentlich geringere Menge an Antikörper erforderlich. Es konnte gezeigt werden, dass der erfindungsgemäße anti-PSCA Antikörper in deutlich geringeren Konzentrationen und mit deutlich höherer Effizienz die spezifische Lyse von PSCA-positiven Tumorzellen vermitteln kann. Dies hat einerseits Kostenvorteile, da der Antikörperverbrauch reduziert werden kann. Andererseits in der therapeutischen Anwendung ist ein verbessertes Targeting vor allem auch von metastasierenden Zellen zu erwarten, sowie aufgrund der geringeren Einsatzmenge mit weniger Nebenwirkungen zu rechnen.

Anhand folgender Figuren und Ausführungsbeispiele soll die Erfindung näher erläutert werden, ohne die Erfindung auf diese zu beschränken.

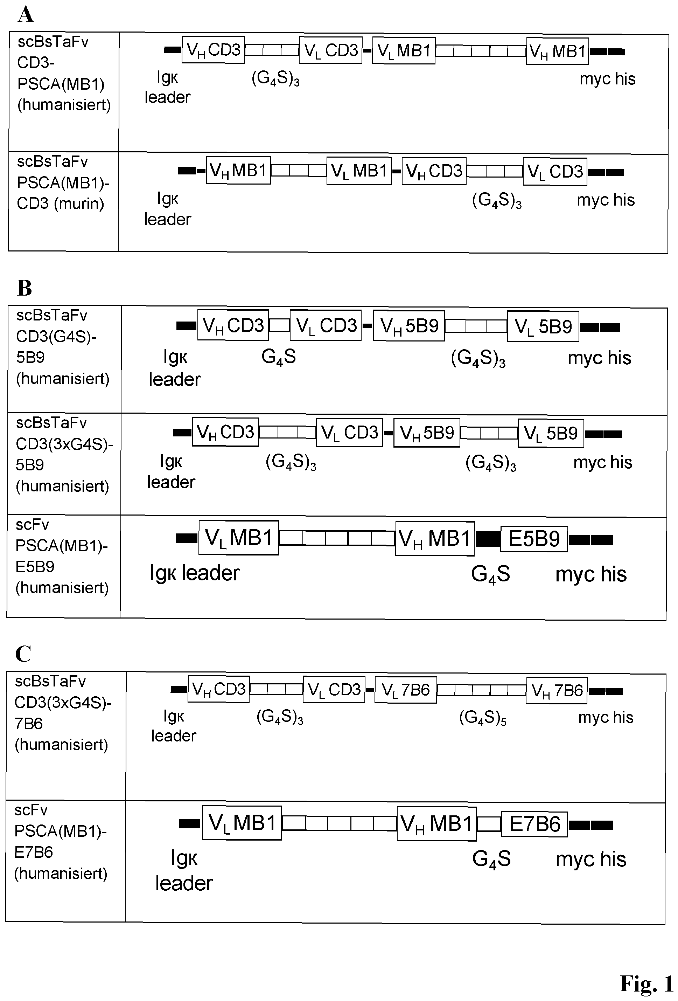

- Fig. 1: Schematische Darstellung unterschiedlicher rekombinanter Antikörper. A) erfindungsgemäßer bispezifischer Antikörper in Form eines scBsTaFv enthaltend eine Bindungseinheit gegen PSCA und eine Bindungseinheit gegen CD3. B) rekombinanter Antikörper gegen PSCA enthaltend ein Peptid-Tag (5B9), zur Verwendung mit dem ebenfalls dargestellten bispezifischen Antikörper in Form eines scBsTaFv enthaltend eine Bindungseinheit gegen CD3 und eine Bindungseinheit gegen die 5B9-Region des humanen La-Proteins (Vergleichsbeispiel). C) rekombinanter Antikörper gegen PSCA enthaltend ein Peptid-Tag (7B6), zur Verwendung mit dem ebenfalls dargestellten bispezifischen Antikörper in Form eines scBsTaFv enthaltend eine Bindungseinheit gegen CD3 und eine Bindungseinheit gegen die 7B6-Region des humanen La-Proteins (Vergleichsbeispiel). Die jeweiligen V_{H} und V_{L} Untereinheiten sind über die Linkerpeptide mit den in den Abbildungen angegebenen Aminosäuresequenzen verknüpft.

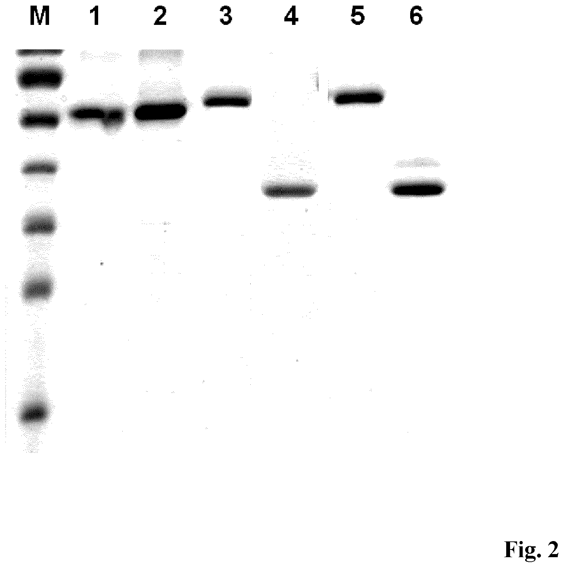

- Fig. 2: SDS-PAGE rekombinanter Antikörper. Spur 1: bispezifischer humanisierter Antikörper CD3-7B6 (scBsTaFv CD3-7B6); Spur 2: bispezifischer humanisierter Antikörper CD3-5B9 (scBsTaFv CD3(G₄S)-5B9); Spur 3: bispezifischer muriner Antikörper CD3-PSCA(7F5) (scBsTaFv CD3-PSCA(7F5)); Spur 4: monospezifischer humanisierter Antikörper scFv PSCA(MB1)-7B6; Spur 5: erfindungsgemäßer bispezifischer humanisierter Antikörper CD3-PSCA(MB1) (scBsTaFv CD3-PSCA(MB1)); Spur 6: monospezifischer humanisierter Antikörper scFv PSCA(MB1)-5B9. M... Marker mit Fragmentgrößen (von oben nach unten): 70 kDa, 55 kDa, 40 kDa, 35 kDa, 25 kDa, 15 kDa.
- Fig. 3: Spezifische Lyse ⁵¹Cr -beladener PC3-PSCA Tumorzellen im Versuch nach Ausführungsbeispiel 4. Die Balken korrespondieren mit folgenden Versuchen: 1 (schwarz) ... humanisierter bispezifischer scBsTaFv CD3-PSCA(MB1) aus Ausführungsbeispiel 1; 2 (weiß) ... muriner bispezifischer scBsTaFv CD3-PSCA(MB1) aus Ausführungsbeispiel 1; 3 (gestreift) ... humanisierter bispezifischer scBsTaFv CD3-PSCA(7F5) (Vergleichsbeispiel); 4 (gepunktet) ... muriner bispezifischer scBsTaFv CD3-PSCA(7F5) (Vergleichsbeispiel).
- Fig. 4: Ausgebildete Tumorfläche nach Transfer von PSCA-positiven Tumorzellen, T Zellen und Antikörper wie in Ausführungsbeispiel 5 beschrieben. A) Effektor-Target-Verhältnis 1:1; B) Effektor-Target-Verhältnis 10:1. Die nummerierten Linien korrespondieren mit folgenden Versuchen: 1 ... bispezifischer Kontrollantikörper anti-CD3xanti-5B9 (Kontrolle); 2 ... monospezifischer humanisierter scFv anti-PSCA(MB1) aus Ausführungsbeispiel 1 (Kontrolle); 3 ... Kontrollversuch ohne Antikörper (Negativkontrolle); 4 ... humanisierter bispezifischer scBsTaFv CD3-PSCA(MB1) aus Ausführungsbeispiel 1 (erfindungsgemäß); 5 ... humanisierter bispezifischer scBsTaFv CD3-PSCA(7F5) wie in Beispiel 3 beschrieben (Vergleichsbeispiel); 6 ... humanisierter bispezifischer scBsDb CD3xPSCA(7F5), der die PSCA-bindenden variablen Regionen des 7F5 Antikörpers enthält (Vergleichsbeispiel).

### Ausführungsbeispiel 1: Herstellung von bispezifischen rekombinanten Antikörpern (scBsTaFv), die spezifisch an PSCA binden (direktes Targeting)

Zum Einsatz für das Targeting von PSCA⁺ Zellen wurde ein bispezifischer Antikörper (single chain bispecific diabody, scBsTaFv) hergestellt, der mit einer Bindungseinheit an PSCA und mit der anderen Bindungseinheit an CD3 bindet. Der bispezifische Antikörper wird hierin auch vereinfacht als CD3-PSCA(MB1) bezeichnet und ist schematisch in Fig. 1A abgebildet.

Die PSCA-bindende Domäne, enthält die variable Region des erfindungsgemäßen anti-PSCA MB1 Antikörpers (muriner anti-PSCA Antikörper: schwere Kette SEQ ID No. 22, leichte Kette SEQ ID No. 20; humanisierter anti-PSCA Antikörper: schwere Kette SEQ ID No. 26, leichte Kette SEQ ID No. 24). Sie dient zur Bindung an die PSCA-positiven Tumorzellen. Die andere Domäne bindet an CD3, einen Bestandteil des T Zell Rezeptor Komplexes, und dient zur Aktivierung von T Zellen. Dadurch wird die Rekrutierung von T Zellen an die PSCA-positiven Zellen ermöglicht und vermittelt auf diese Weise die spezifische Lyse der PSCA-positiven Zellen durch die T Zellen.

Für die Generierung der monoklonalen anti-PSCA MB1 Antikörper wurden H-2d positive C3Hx Balb/c F1-Mäuse mit P815-Zellen immunisiert, die PSCA rekombinant auf der Oberfläche exprimierten. Durch die Fusion von Milzzellen und Myelomzellen entstanden Hybridomazellen, die monoklonale anti-PSCA Antikörper sezernierten. Nach Einzellklonierung dieser Hybridomazellen konnte der Klon MB1 selektioniert werden. Für die Generierung von rekombinanten anti-PSCA MB1 Antikörpern wurden die Nukleinsäuresequenzen der variablen Domäne der schweren (V_{H}) und leichten (V_{L}) Antikörperkette identifiziert. Dafür wurde zunächst die mRNA aus den anti-PSCA MB1 sezernierenden Hybridomazellen isoliert und in cDNA umgeschrieben. Anschließend wurde die variable Domäne der schweren Kette vom Isotyp IgG1 mit degenerierten Primern (Primerpaar SEQ ID No. 90 und 91) sowie die variable Domäne der leichten κ Kette mit Hilfe degenerierter Primer (Primerpaar SEQ ID No. 92 und 93) amplifiziert. Die PCR-Produkte wurden jeweils in den Vektor pGEM T-easy subkloniert und sequenziert.

Für die Klonierung des murinen Einzelkettenfragments (scFv) des anti-PSCA MB1 Antikörpers (hierin vereingacht als "scFv MB1" bezeichnet), bei dem die variable Region der schweren Kette über drei Glycin-Serin Linker (G₄S, SEQ ID No. 131) mit der variablen Region der leichten Kette verknüpft ist, wurde die Nukleinsäuresequenz der variablen Region der schweren Kette des anti-PSCA MB1 Antikörpers mit Hilfe des Primerpaares gemäß SEQ ID No. 94 und 95 amplifiziert. Die Nukleinsäuresequenz der variablen Region der leichten Kette des anti-PSCA MB1 Antikörpers wurde mit Hilfe des Primerpaares gemäß SEQ ID No. 96 und 97 amplifiziert. Die amplifizierten Nukleinsäuren wurden mittels overlap PCR zum scFv PSCA MB1 fusioniert und über SfiI und NotI in den eukaryotischen Expressionsvektor pSecTag2B kloniert (der Expressionsvektor wird hierin auch als "pSecTag2B_scFv MB1 murin" bezeichnet).

Für die Klonierung des bispezifischen Tandemantikörpers (scBsTaFv), der gegen PSCA und CD3 gerichtet ist und der die CDR Regionen des murinen MB1 Antikörpers enthält, wurde die Nukleinsäuresequenz eines murinen anti-CD3 scFv mit einem Primerpaar gemäß SEQ ID No. 98 und 99 amplifiziert und über ApaI 3'-seitig vom murinen scFv MB1 in den zuvor produzierten Expressionsvektor "pSecTag2B_scFv MB1 murin" kloniert, so dass der Vektor "pSecTag2B_ scBsTaFv PSCA(MB1)-CD3 murin" entstand.

Für die Klonierung des bispezifischen Tandemantikörpers (scBsTaFv), der gegen PSCA und CD3 gerichtet ist und der die CDR Regionen des humanisierten MB1 Antikörpers enthält, wurden die Gerüstregionen (FWR) der PSCA MB1 und CD3 Antikörper humanisiert. Dafür wurden die FWR der der variablen murinen Antikörperdomänen gegen die humanen FWR-Sequenzen eines hoch homologen humanen IgG1 ersetzt. Darüber hinaus wurden die humanisierten Antikörpersequenzen hinsichtlich ihrer Expression und Sekretion durch humane Zelllinien optimiert. Für die Humanisierung des murinen scFv CD3 bzw. scFv MB1 wurden zunächst die humanisierten V_{H}- und V_{L}-Sequenzen einzeln wie vorstehend beschrieben amplifiziert und anschließend über PCR zusammengefügt. Die Humanisierung der V_{H}- bzw. V_{L}-Sequenz erfolgte dabei durch die Zusammenlagerung von überlappenden Oligonukleotiden und anschließender Amplifikation der humanisierten V_{H}- bzw. V_{L}-Nukleinsäuresequenz. Um die Sequenzen der überlappenden Oligonukleotide festlegen zu können, wurde zunächst die murine V_{H}- bzw. V_{L}-Sequenz gegen eine humane IgG Datenbank (NCBI IgBlast) abgeglichen, der humane IgG mit der größten Homologie identifiziert und letztendlich die humanisierte V_{H}- bzw. V_{L}-Sequenz theoretisch erstellt und die überlappenden Oligonukleotide synthetisiert. Es wurden verschiedene bispezifische Antikörper konstruiert, bei denen unterschiedlich lange Linkerpeptide eingesetzt wurden.

### Humanisierung des anti-CD3 Antikörpers

Für die Humanisierung der variablen Region der schweren Kette des anti-CD3 Antikörpers wurden Oligonukleotide gemäß SEQ ID No. 100 bis 105 zusammengelagert. Anschließend wurde die humanisierte Nukleinsäuresequenz der variablen Region der schweren Kette des anti-CD3 Antikörpers (mit einem G₄S-Linkerpeptid, SEQ ID No. 132) mit Hilfe des Primerpaares gemäß SEQ ID No. 105 und 106 amplifiziert. Für die Humanisierung der variablen Region der leichten Kette des anti-CD3 Antikörpers wurden Oligonukleotide gemäß SEQ ID No. 107 bis 112 zusammengelagert. Die humanisierte Nukleinsäuresequenz der variablen Region der leichten Kette des anti-CD3 Antikörpers wurde mit dem Primerpaar gemäß SEQ ID No. 112 und 113 amplifiziert.

Für die Generierung des humanisierten scFv des anti-CD3 Antikörpers, bei dem die variable Region der schweren Kette über einen Glycin-Serin Linker (G₄S) mit der variablen Region der leichten Kette verknüpft ist, wurde zuerst die humanisierte Nukleinsäuresequenz der variablen Region der schweren Kette des anti-CD3 Antikörpers über SfiI und BamHI in den Expressionsvektor pSecTag2B kloniert und anschließend über BamHI und NotI die humanisierte Nukleinsäuresequenz der variablen Region der leichten Kette des anti-CD3 Antikörpers stromabwärts davon in denselben Expressionsvektor gesetzt.

Für die Generierung des humanisierten scFv des anti-CD3 Antikörpers, bei dem die variable Region der schweren Kette über drei Glycin-Serin Linker (G₄S) mit der variablen Region der leichten Kette verknüpft ist, wurde zuerst die humanisierte Nukleinsäuresequenz der variablen Region der schweren Kette des anti-CD3 Antikörpers mit drei Glycin-Serin Linkern (G₄S) hergestellt. Dafür wurde die humanisierte Nukleinsäuresequenz der variablen Region der schweren Kette des anti-CD3 Antikörpers (mit drei G₄S-Linkerpeptiden) mit Hilfe des Primerpaares gemäß SEQ ID No. 105 und 114 amplifiziert und über SfiI und BamHI in den Expressionsvektor pSecTag2B kloniert und anschließend über BamHI und NotI die humanisierte Nukleinsäuresequenz der variablen Region der leichten Kette des anti-CD3 Antikörpers stromabwärts davon in denselben Expressionsvektor gesetzt.

### Humanisierung des anti-PSCA MB1 Antikörpers

Für die Humanisierung der variablen Region der leichten Kette des anti-PSCA MB1 Antikörpers wurden Oligonukleotide gemäß SEQ ID No. 115 bis 118 zusammengelagert. Die humanisierte Nukleinsäuresequenz der variablen Region der leichten Kette des anti-PSCA MB1 Antikörpers wurde mit dem Primerpaar gemäß SEQ ID No. 119 und 120 amplifiziert.

Für die Humanisierung der variablen Region der schweren Kette des anti-PSCA MB1 Antikörpers wurden Oligonukleotide gemäß SEQ ID No. 121 bis 125 zusammengelagert. Die humanisierte Nukleinsäuresequenz der variablen Region der schweren Kette des anti-PSCA MB1 Antikörpers wurde mit dem Primerpaar gemäß SEQ ID No. 126 und 127 amplifiziert.

### Herstellung des bispezifischen humanisierten Antikörpers scBsTaFv CD3-PSCA(MB1)

Schließlich wurden die humanisierten Nukleinsäuresequenzen der variablen Region der schweren Kette und der variablen Region der leichten Kette des MB1 Antikörpers mittels overlap PCR unter Verwendung der Primer gemäß SEQ ID No. 128 und129 zusammengelagert und amplifiziert. Die so generierte Nukleinsäuresequenz codierend für den humanisierten scFv MB1 (in der Organisation V_{L}-G₄SG₄SGASAAG₄SG₄S-V_{H}, Linkerpeptid gemäß SEQ ID No. 130) wurde über XhoI und ApaI stromabwärts von dem oben beschriebenen humanisierten scFv des anti-CD3 Antikörpers (mit drei G₄S-Linkerpeptiden) kloniert, wobei der Expressionsvektor "pSecTag2B_ scBsTaFv PSCA(MB1)-CD3 human" entstand.

Zur Expression des bispezifischen humanisierten Antikörpers gemäß Fig. 1 A wurden Hek293T Zellen mit dem Expressionsvektor transfiziert und die sezernierten Antikörper aus den Zellkulturüberständen mittels Nickel-Affinitätschromatographie gegebenenfalls in Kombination mit einer fraktionierten Ammoniumsulfatfällung aufgereinigt. Fig. 2 Spur 5 zeigt eine SDS-Gelelektrophorese Aufnahme des aufgereinigten bispezifischen Antikörpers.

### Vergleichsbeispiel: Herstellung von bispezifischen rekombinanten Antikörpern, die spezifisch an PSCA binden (modulares Targeting)

Zur Bereitstellung einer pharmazeutischen Zusammensetzung ("modulares Targeting System 1", schematisch in Fig. 1 B dargestellt) wurden folgende rekombinante Antikörper hergestellt:
- rekombinanter anti-PSCA Antikörper enthaltend ein Peptid gemäß SEQ ID No. 75 (hierin auch "E5B9"). Dieser Antikörper wird im Folgenden als scFv PSCA(MB1)-E5B9 bezeichnet.
- bispezifischer Antikörper (scBsTaFv), der gegen CD3 und das Peptid gemäß SEQ ID No. 75 gerichtet ist. Das gegen CD3 gerichtete Paratop umfasst die folgenden Aminosäuresequenzen der variablen Regionen: schwere Kette SEQ ID No. 65, leichte Kette SEQ ID No. 57. Das gegen das Peptid gemäß SEQ ID No. 75 gerichtete Paratop umfasst folgende Aminosäuresequenzen der hypervariablen Regionen der variablen Regionen: leichte Kette CDR1 SEQ ID No. 78, CDR2 SEQ ID No. 79, CDR3 SEQ ID No. 80, schwere Kette CDR1 SEQ ID No. 81, CDR2 SEQ ID No. 82, CDR3 SEQ ID No. 83. Dieser Antikörper wird im Folgenden als scBsTaFv CD3-5B9 bezeichnet. Es wurden zwei unterschiedliche scBsTaFv CD3-5B9 hergestellt, die sich lediglich voneinander in dem Linkerpeptid zwischen VH und VL des anti-CD3Antikörpers unterscheiden (siehe Fig. 1B).

### Klonierung des humanisierten scFv PSCA(MB1)-ESB9:

Für die Generierung des humanisierten scFv PSCA(MB1) mit E5B9-Epitop am C-Terminus (MB1 [V_{L}-G₄SG₄SGASAAG₄SG₄S-MB1 V_{H}]-[G₄S-E5B9], dargestellt in Fig. 1B unten) wurde der in Ausführungsbeispiel 1 beschriebene humanisierte scFv PSCA(MB1) (MB1 V_{L}-G₄SG₄SGASAAG₄SG₄S-MB1 V_{H}-G₄S, Linkerpeptide gemäß SEQ ID No. 130 und 132) mittels der Primer gemäß SEQ ID No. 134 und 135 amplifiziert und anschließend über SfiI und NotI in pSecTag2B kloniert. Nach Zusammenlagerung der Oligonukleotide gemäß SEQ ID No. 136 und 137 wurde die Nukleinsäuresequenz für E5B9 über NotI und XhoI 3'-seitig vom humanisierten scFv PSCA(MB1) einkloniert, wobei das Konstrukt "pSecTag2B_ scFv PSCA(MB1)-E5B9 humanisiert" entstand.

### Klonierung zweier an T-Zellen und an das E5B9-Peptid bindender Effektormodule "scBsTaFv CD3(G₄S)-5B9" und "scBsTaFv CD3(3×G₄S)-5B9"

Wie schematisch in Fig. 1B dargestellt ist, wurden zum Aufbau des modularen Targetingsystems I umfassend das scFv PSCA(MB1)-5B9 zwei sogenannte Effektormodule (bispezifische Antikörper scBsTaFv CD3-5B9) hergestellt, die sich in der Anzahl der Glycin-Serin (G₄S) Elemente des Linkerpeptids zwischen der V_{H} und V_{L}-Kette der anti-CD3 Domäne unterscheiden und deshalb als "scBsTaFv CD3(G₄S)-5B9" bzw. "scBsTaFv CD3(3×G₄S)-5B9" bezeichnet wurden.
- Klonierung des "scBsTaFv CD3(G₄S)-5B9" humanisiert (Schema siehe Fig. 1B oben):
   Für die Humanisierung der variablen Region der schweren Kette des 5B9 Antikörpers (spezifisch gegen das Peptid E5B9 gerichtet) wurden Oligonukleotide gemäß SEQ ID No. 138 bis 142 zusammengelagert. Anschließend wurde die humanisierte Nukleinsäuresequenz der variablen Region der schweren Kette des 5B9 Antikörpers (mit einem G₄S-Linkerpeptid, SEQ ID No. 132) mit Hilfe des Primerpaares gemäß SEQ ID No. 143 und 144 amplifiziert. Für die Humanisierung der variablen Region der leichten Kette des 5B9 Antikörpers wurden Oligonukleotide gemäß SEQ ID No. 145 bis 149 zusammengelagert. Die humanisierte Nukleinsäuresequenz der variablen Region der leichten Kette des 5B9 Antikörpers wurde mit dem Primerpaar gemäß SEQ ID No. 150 und 151 amplifiziert.
   Schließlich wurden die humanisierten Nukleinsäuresequenzen für 5B9 V_{H} und 5B9 V_{L} mittels overlap PCR unter Verwendung der Primer gemäß SEQ ID No. 152 und 153 zusammengelagert und amplifiziert. Die so generierte Nukleinsäuresequenz für den humanisierten scFv 5B9 (V_{H}-3×G₄S-V_{L}) wurde über XhoI und ApaI stromabwärts vom humanisierten scFv CD3 V_{H}-G₄S-V_{L} in den "pSecTag2B_scFv CD3 V_{H}-G₄S-V_{L}" humanisiert kloniert, wobei der Vektor "pSecTag2B_scBsTaFv CD3(G₄S)-5B9 humanisiert" entstand.
- Klonierung des "scBsTaFv CD3(3×G₄S)-5B9" humanisiert (Schema siehe Fig. 1B Mitte):
   Die humanisierte Sequenz für scFv 5B9 (V_{H}-3×G₄S-V_{L}) wurde über XhoI und ApaI stromabwärts vom humanisierten scFv CD3 (V_{H}-3×G₄S-V_{L}) in den "pSecTag2B_scFv CD3 V_{H}-3×G₄S-V_{L}" humanisiert kloniert, wobei der Vektor "pSecTag2B_scBsTaFv CD3(3×G₄S)-5B9 humanisiert" entstand.

Zur Bereitstellung einer pharmazeutischen Zusammensetzung ("modulares Targeting System 2", schematisch in Fig. 1 C dargestellt) wurden folgende rekombinante Antikörper hergestellt:
- rekombinanter anti-PSCA Antikörper enthaltend ein Peptid gemäß SEQ ID No. 76 (hierin auch als "E7B6" bezeichnet). Dieser Antikörper wird im Folgenden als scFv PSCA(MB1)-E7B6 bezeichnet.
- bispezifischer Antikörper (scBsTaFv), der gegen CD3 und das Peptid gemäß SEQ ID No. 76 gerichtet ist. Das gegen CD3 gerichtete Paratop umfasst die folgenden Aminosäuresequenzen der variablen Regionen: schwere Kette SEQ ID No. 65, leichte Kette SEQ ID No. 57. Das gegen das Peptid gemäß SEQ ID No. 76 gerichtete Paratop umfasst folgende Aminosäuresequenzen der hypervariablen Regionen der variablen Regionen: leichte Kette CDR1 SEQ ID No. 84, CDR2 SEQ ID No. 85, CDR3 SEQ ID No. 86, schwere Kette CDR1 SEQ ID No. 87, CDR2 SEQ ID No. 88, CDR3 SEQ ID No. 89. Dieser Antikörper wird im Folgenden als scBsTaFv CD3-7B6 bezeichnet.

### Klonierung des humanisierten scFv PSCA(MB1)-E7B6:

Für die Generierung des humanisierten scFv PSCA(MB1) mit E7B6-Epitop am C-Terminus MB 1 [V_{L}-G₄SG₄SGASAAG₄SG₄S-MB1 V_{H}]-[G₄S-E7B6], dargestellt in Fig. 1C unten) wurde zunächst die Zusammenlagerung der Oligonukleotide gemäß SEQ ID No. 154 und 155 durchgeführt und anschließend die Klonierung der E7B6-Nukleinsäuresequenz über NotI und XhoI 3'-seitig in "pSecTag2B₋ scFv PSCA(MB1) humanisiert" (V_{L}-G₄SG₄SGASAAG₄SG₄S-V_{H}-G₄S, analog zum oben beschriebenen Konstrukt mit dem E5B9 Peptid), wobei der Vektor "pSecTag2B_ scFv PSCA(MB1)-E7B6 humanisiert" entstand.

### Klonierung eines an T-Zellen und an das E7B6-Peptid bindenden Effektormoduls "scBsTaFv CD3(3×G₄S)-7B6" humanisiert:

Für die Humanisierung der variablen Region der schweren Kette des 7B6 Antikörpers (spezifisch gegen das Peptid E7B6 gerichtet) wurden überlappende Oligonukleotide gemäß SEQ ID No. 156 bis 160 zusammengelagert. Anschließend wurde die humanisierte Nukleinsäuresequenz der variablen Region der schweren Kette des 7B6 Antikörpers (mit einem G₄S-Linkerpeptid, SEQ ID No. 132) mit Hilfe des Primerpaares gemäß SEQ ID No. 161 und 162 amplifiziert. Für die Humanisierung der variablen Region der leichten Kette des 7B6 Antikörpers wurden Oligonukleotide gemäß SEQ ID No. 163 bis 167 zusammengelagert. Die humanisierte Nukleinsäuresequenz der variablen Region der leichten Kette des 7B6 Antikörpers wurde mit dem Primerpaar gemäß SEQ ID No. 168 und 169 amplifiziert.

Schließlich wurden die humanisierten Nukleinsäuresequenzen für 7B6 V_{H} und 7B6 V_{L} mittels overlap PCR unter Verwendung der Primer gemäß SEQ ID No. 170 und 171 zusammengelagert und amplifiziert. Die so generierte Nukleinsäuresequenz für den humanisierten scFv 7B6 (V_{H}-3×G₄S-V_{L}) wurde über XhoI und ApaI stromabwärts vom humanisierten scFv CD3 V_{H}-G₄S-V_{L} in den "pSecTag2B_scFv CD3 V_{H}-G₄S-V_{L}" humanisiert kloniert, wobei der Vektor "pSecTag2B_scBsTaFv CD3(G₄S)-7B6 humanisiert" entstand.

Zur Expression der einzelnen Fusionsproteine der modularen Targeting Systeme 1 und 2 wurden Hek293T Zellen mit den Expressionsvektoren transfiziert und die sezernierten rekombinanten scFv (scFv PSCA-E5B9 oder scFv PSCA-E7B6) und die bispezifischen Antikörper (scBsTaFv CD3(G₄S)-5B9 bzw. scBsTaFv CD3(3×G₄S)-5B9 und scBsTaFv CD3-7B6) aus dem Zellkulturüberstand mit Hilfe von Affinitätschromatographie über Ni-NTA Agarose (Qiagen, Hilden, Deutschland) (gegebenenfalls in Kombination mittels einer fraktionierten Ammoniumsulfatfällung) aufgereinigt. Per SDS-Page und Immunoblot wurde die Reinheit und Stabilität der rekombinanten Antikörperderivate nachgewiesen (Fig. 2)

### Bestimmung der Dissoziationskonstante der Bindung an PSCA

Die Ermittlung der Affinitätskonstante für die jeweilige anti-PSCA Domäne der rekombinanten, bispezifischen Antikörper mit dem anti-PSCA MB1 Antikörper (erfindungsgemäß) und dem anti-PSCA 7F5 Antikörper (Vergleichsbeispiel, Antikörper aus [Feldmann 2011]) basierte auf einer durchflusszytometrischen Analyse der Bindung an PSCA-positive PC3 Zellen.

Für die Generierung der Bindungskurven der rekombinanten anti-PSCA Antikörperdomänen wurden jeweils 2×10⁵ PSCA-positive Zellen mit 100 µl der bispezifischen Antikörper (MB1 und 7F5) für 1 h bei 4°C inkubiert. Die Antikörper wurden jeweils in folgenden Konzentrationen eingesetzt:

| Antikörpermenge je Ansatz in ng | Antikörper-Konzentration in pmol/l |
|---|---|
| 1000 | 90000 |
| 100 | 9000 |
| 50 | 4500 |
| 10 | 900 |
| 5 | 450 |
| 1 | 90 |
| 0,5 | 45 |
| 0,1 | 9 |
| 0,05 | 4,5 |
| 0,01 | 0,9 |
| 0,001 | 0,09 |

Um die spezifische Bindung der rekombinanten CD3-PSCA Antikörper nachzuweisen, wurde ein Maus-anti-c-myc IgG-FITC Nachweisantikörper (AbD Serotec, Düsseldorf, Deutschland) verwendet, der nach Abschluss des ersten Färbeschrittes für 30 min bei 4°C mit den anti-PSCA Antikörpermarkierten PSCA-positiven Zellen inkubiert wurde. Als Negativkontrolle wurde eine Probe mitgeführt, in der PSCA-positive Zellen nur mit dem Nachweisantikörper Maus-anti-c-myc IgG-FITC angefärbt wurde. Die Zellen wurden an einem BD FACS Calibur Flow Cytometer (BD Biosciences Pharmingen, Heidelberg, Deutschland) analysiert und die Daten wurden mit Hilfe der Software WinMDI 2.8 (Joseph Trotter, La Jolla, CA USA) ausgewertet.

Zur Auswertung wurden die ermittelten MFI-Werte (mittlere Fluoreszenzintensitätswerte) gegen die getesteten Antikörperkonzentrationen aufgetragen und jeweils eine Trendlinie vom polynomischen Regressionstyp zweiter Ordnung berechnet. Um die Affinitätskonstante zu ermitteln, wurde eine Trendlinie vom polynomischen Regressionstyp zweiter Ordnung berechnet und eingefügt. Die daraus resultierende Bindungskurve diente als Grundlage zur Berechnung der Affinitätskonstanten K_{D}, welche als diejenige Konzentration definiert ist, die beim 50%-Wert des maximalen MFI-Wertes und somit bei halbmaximaler Sättigung der Bindung erreicht wird. Um diese Konstante zu berechnen, wurde zunächst die erste Ableitung der Bindungskurve, welche einer quadratischen Funktion folgt, gebildet. Um ausgehend von dieser Gleichung das Maximum der Funktion zu ermitteln, wurde die erste Ableitung "Null" gesetzt und nach x aufgelöst. Durch Einsetzen des erhaltenen x-Wertes in die Ausgangsgleichung wurde das Maximum der Funktion yₘₐₓ und somit yₘₐₓ/2, welches dem MFI-Wert bei halbmaximaler Absättigung der Bindungsstellen entspricht, errechnet. Da der K_{D}-Wert dem x-Wert bei yₘₐₓ/2 entspricht, kann dieser durch Einsetzen des yₘₐₓ/2-Wertes in die quadratische Funktion der Bindungskurve und Umstellen der Gleichung nach x abschließend kalkuliert werden

Folgende K_{D}-Werte wurden auf diese Weise für die Bindung an PSCA ermittelt:

| | K_{D} |
|---|---|
| scBsTaFv CD3×PSCA(MBl) | 6,3 × 10⁻⁷ |
| scBsTaFv CD3xPSCA(7F5) | 2,3 × 10⁻⁶ |

Es konnte gezeigt damit werden, dass die Affinität des erfindungsgemäßen anti-PSCA Antikörper zum Antigen PSCA eine Größenordnung höher ist, als die des aus dem Stand der Technik bekannten Antikörpers 7F5.

### Spezifische Lyse von PSCA+ Zellen mit bispezifischen anti-PSCA Antikörpern

Im Chromfreisetzungstest wurden voraktivierte PBMCs 5×10⁴ mit 5×10^{3 51}Cr-beladene PC3-PSCA Tumorzellen (Effektor-Target-Verhältnis = 10:1) in An- und Abwesenheit der rekombinanten Antikörper in RPMI Medium in einem Gesamtvolumen von 200µl kokultiviert. Dazu wurden die erfindungsgemäßen Antikörper aus Beispiel 1 eingesetzt (sowohl in muriner als auch in humanisierter Form). Als Vergleichsbeispiel wurde der aus dem Stand der Technik bekannter muriner anti-PSCA(7F5) Antikörper in einem gleichartig aufgebauten bispezifischen Antikörper eingesetzt. Die variablen Regionen des 7F5 Antikörpers entsprechen: schwere Kette SEQ ID No. 48, leichte Kette SEQ ID No. 50. Es wurden Versuche mit folgenden konkreten Antikörperkonstrukten durchgeführt:
1. humanisierter bispezifischer scBsTaFv CD3-PSCA(MB1) aus Ausführungsbeispiel 1
2. muriner bispezifischer scBsTaFv CD3-PSCA(MB1) aus Ausführungsbeispiel 1
3. humanisierter bispezifischer scBsTaFv CD3-PSCA(7F5) (Vergleichsbeispiel)
4. muriner bispezifischer scBsTaFv CD3-PSCA(7F5) (Vergleichsbeispiel)

Nach 20h Inkubation bei 37°C im Brutschrank wurde das ins Medium freigesetzte Chrom gemessen. Die spezifische Lyse wurde wie folgt berechnet: Spezifische Lyse in % = [freigesetztes 51Cr - spontan freigesetztes 51Cr (Minimum)]/[maximal freigesetztes 51Cr (Maximum) - Minimum] × 100%

Die Ergebnisse des *in vitro* Versuchs sind graphisch in Fig. 3 dargestellt. Dargestellt ist der prozentuale Anteil lysierter PC3 Zellen an den insgesamt eingesetzten PC3 Zellen (y-Achse). Während die erfindungsgemäßen Antikörper der Versuchsgruppen 1 und 2 bereits bei Mengen von weniger als 1 ng eine starke Lyse der PSCA-positiven Zellen zeigen, ist bei dem bekannten 7F5 Antikörper in einem vergleichbaren Konstrukt (Versuchsgruppen 3 und 4) eine detektierbare Lyse erst beim Einsatz mehrerer ng des Antikörpers festzustellen. Maximal wurde eine Lyse von etwa 60% der eingesetzten PC3 Zellen festgestellt. Mit den erfindungsgemäßen Antikörpern konnten bereits beim Einsatz von einer deutlich geringeren Antikörpermenge eine Lyse von etwa 80 % der eingesetzten PC3 Zellen festgestellt werden. Maximal konnten mehr als 90 % der eingesetzten PC3 Zellen mit einem erfindungsgemäßen Antikörper (Versuchsgruppe 1) lysiert werden.

### Hemmung des Tumorwachstums durch scBsTaFv CD3-PSCA(MB1) humanisiert in vivo

Zum Nachweis der Wirksamkeit des erfindungsgemäßen anti-PSCA MB1 Antikörpers wurde der Effekt eines davon abgeleiteten bispezifischen Antikörpers scBsTaFv CD3-PSCA(MB1), der nach Ausführungsbeispiel 1 hergestellt wurde (schematisch dargestellt in Fig. 1 A, oben), im murinen Tumormodell getestet. Als Modellorganismus wurden Nacktmäuse eingesetzt, die nach Transfer von PSCA-positiven PC3 Tumorzellen Tumoren ausbilden. Im Versuch wurden PSCA-positiven Tumorzellen in Kombination mit T Zellen und einem Antikörper transferiert.

Folgende Antikörper wurden dafür eingesetzt, wobei je Maus 10 µg Antikörper injiziert wurden:
(1) bispezifischer Kontrollantikörper anti-CD3xanti-5B9 (Kontrolle)
(2) monospezifischer humanisierter scFv anti-PSCA(MB1) aus Ausführungsbeispiel 1 (Kontrolle)
(3) Kontrollversuch ohne Antikörper (Negativkontrolle)
(4) humanisierter bispezifischer scBsTaFv CD3-PSCA(MB1) aus Ausführungsbeispiel 1
(5) humanisierter bispezifischer scBsTaFv CD3-PSCA(7F5) (Vergleichsbeispiel)
(6) humanisierter bispezifischer scBsDb CD3xPSCA(7F5), der die PSCA-bindenden variablen Regionen des 7F5 Antikörpers enthält (Vergleichsbeispiel)

Die Wirkung der bispezifischen Antikörper beruht auf der Rekrutierung der CD3-positiven T-Zellen zu den PSCA-positiven Tumorzellen und der dadurch vermittelten Lyse der Tumorzellen.

In einem ersten Versuch wurden 5×10³ PSCA-positive Tumorzellen in jeweils 8 Nacktmäuse pro Versuchsgruppe und 5×10³ T-Zellen transferiert (Effektor-Target-Verhältnis 1:1). In den Kontrollgruppen (1) und (3) wurde in Abwesenheit eines anti-PSCA Antikörpers rapides Tumorwachstum festgestellt. Auch der Kontrollversuch (2), bei dem der monospezifische scFv anti-PSCA(MB1) injiziert wurde, zeigte rapides Tumorwachstum. Dadurch lässt sich ein cytotoxischer Effekt des monospezifischen Antikörpers ausschließen und die Wirksamkeit allein dem bispezifischen Konstrukt zugeschrieben werden. Die Tumorfläche der gebildeten Tumoren wurde 25 Tage nach Zelltransfer ermittelt. Die drei Kontrollgruppen zeigten keinen signifikanten Unterschied der Tumorfläche (Fig. 4A).

Bei Transfer der bispezifischen Vergleichsantikörpers, die die variablen Regionen des anti-PSCA 7F5 Antikörpers enthalten (Vergleichsgruppen (5) in Form eines Tandemantikörpers und (6) in Form eines Diabodys) wurde ebenfalls ein rapides Tumorwachstum festgestellt (Daten nicht dargestellt). Auch in einem zweiten Versuch, bei der die Tumorzellen in einem Effektor-Target-Verhältnis von 10:1 (also bei 10-facher Menge T Zellen; Transfer von 5×10³ PSCA-positiven Tumorzellen und 5×10⁴ T Zellen) wurde ein rapides Tumorwachstum festgestellt, dass keinen statistisch signifikanten Unterschied zur Kontrollgruppe (1) zeigte (Fig. 4B). Das Tumorwachstum wurde durch die anti-PSCA 7F5 Antikörper nicht gehemmt, sondern lediglich leicht verzögert. Auch bei T Zell Überschuss kann somit keine *in vivo* Hemmung des Tumorwachstums durch den anti-PSCA 7F5 Antikörper festgestellt werden.

Der Transfer des erfindungsgemäßen bispezifischen Antikörpers scBsTaFv CD3-PSCA(MB1) aus Ausführungsbeispiel 1 bei einem Effektor-Target-Verhältnis 1:1 konnte das Tumorwachstum signifikant verringern. Während 25 Tage nach Zelltransfer in 2 der 8 Versuchstiere Tumoren mit einer deutlich geringen Fläche (Durchmesser etwa 2 mm) auftraten, blieben 6 der 8 Versuchstiere tumorfrei (Fig 4A). Der erfindungsgemäße anti-PSCA MB1 Antikörper (Versuchsgruppe (4)) ist somit dem vergleichbar aufgebautem anti-PSCA 7F5 Antikörper (Versuchsgruppe (5)) hinsichtlich der *in vivo* Wirksamkeit unter identischen Versuchsbedingungen deutlich überlegen.

### Zitierte Nichtpatentliteratur

Feldmann A, Stamova S, Bippes CC, Bartsch H, Wehner R, Schmitz M, Temme A, Cartellieri M, Bachmann M. Retargeting of T cells to prostate stem cell antigen expressing tumor cells: comparison of different antibody formats. Prostate. 2011 Jun 15;71(9):998-1011. doi: 10.1002/pros.21315. Epub 2010 Dec 28.

Gu Z, Yamashiro J, Kono E, Reiter RE. Anti-prostate stem cell antigen monoclonal antibody 1G8 induces cell death in vitro and inhibits tumor growth in vivo via a Fc-independent mechanism. Cancer Res. 2005 Oct 15;65(20):9495-500.

Lepin EJ et al. An Affinity matured minibody for PET imaging of prostate stem cell antigen (PSCA)-expressing tumors", Europ J Nucl Med and Mol Imaging. 2010. April 1: 37 (8): 1529-1538.

Morgenroth A, Cartellieri M, Schmitz M, Günes S, Weigle B, Bachmann M, Abken H, Rieber EP, Temme A. Targeting of tumor cells expressing the prostate stem cell antigen (PSCA) using genetically engineered T-cells. Prostate. 2007 Jul 1;67(10):1121-31.

Reiter, R.E., Gu, Z., Watabe, T., Thomas, G., Szigeti, K., Davis, E., Wahl, M., Nisitani, S., Yamashiro, J., Le Beau, M.M., Loda, M. und Witte, O.N. Prostate stem cell antigen: A cell surface marker overexpressed in prostate cancer. Proc Natl Acad Sci USA. 95(4): 1735-40 (1998)

Roehl, K.A., M. Han, C. G. Ramos, J. A. V. Antenor, and W. J. Catalona. Cancer progression and survival rates following anatomical radical retropubic prostatectomy in 3,478 consecutive patients: long-term results. J Urol, 172(3):910-4, Sep 2004.

Thomas-Kaskel, A.-K., R. Zeiser, R. Jochim, C. Robbel, W. Schultze-Seemann, C. F. Waller, and H. Veelken. Vaccination of advanced prostate cancer patients with PSCA and PSA peptide-loaded dendritic cells induces DTH responses that correlate with superior overall survival. Int J Cancer, 119(10):2428-34, Nov 2006.

## Patentansprüche

1. Antikörper umfassend mindestens zwei unterschiedliche Bindungseinheiten, wobei
i. mindestens eine der Bindungseinheiten an Prostata-spezifisches Stammzellantigen (PSCA) bindet, wobei
- eine variable Region der leichten Kette die Aminosäure-Sequenzen SEQ ID No. 1, SEQ ID No. 2 und SEQ ID No. 3 umfasst und
- eine variable Region der schweren Kette die Aminosäure-Sequenzen SEQ ID No. 4, SEQ ID No. 5 und SEQ ID No. 6 umfasst;
ii. mindestens eine weitere der Bindungseinheiten gegen CD3 gerichtet ist.

2. Antikörper nach Anspruch 1, wobei die gegen CD3 gerichtete Bindungseinheit enthält:
- eine variable Region der schweren Kette enthaltend die Aminosäuresequenzen SEQ ID No. 66, SEQ ID No. 67 und SEQ ID No. 68 und
- eine variable Region der leichten Kette enthaltend die Aminosäuresequenzen SEQ ID No. 58, SEQ ID No. 59 und SEQ ID No. 60.

3. Antikörper nach Anspruch 1 oder 2, dessen variable Regionen humanisiert sind.

4. Antikörper nach einem der Ansprüche 1 bis 3, wobei die gegen PSCA gerichtete Bindungseinheit
(i) eine variable Region der schweren Kette gemäß SEQ ID No. 26 oder eine Aminosäure-Sequenz mit mindestens 80% Sequenzidentität zu SEQ ID No. 26 umfasst und
(ii) eine variable Region der leichten Kette gemäß SEQ ID No. 24 oder eine Aminosäure-Sequenz mit mindestens 80% Sequenzidentität zu SEQ ID No. 24 umfasst.

5. Antikörper nach einem der Ansprüche 1 bis 4, wobei die gegen CD3 gerichtete Bindungseinheit eine variable Region der leichten Kette gemäß SEQ ID No. 57 und eine variable Region der schweren Kette gemäß SEQ ID No. 65 umfasst.

6. Antikörper nach einem der Ansprüche 1 bis 5 in Form eines Diabody, Triabody oder Tetrabody.

7. Antikörper nach einem der Ansprüche 1 bis 6, wobei die gegen PSCA gerichtete Bindungseinheit ein scFv-Fragment mit der folgenden Struktur V_{L}-G₄SG₄SGASAAG₄SG₄S-V_{H} ist, wobei V_{L} die variable Region der leichten Kette und V_{H} die variable Region der schweren Kette ist und wobei G₄SG₄SGASAAG₄SG₄S ein Linkerpeptid gemäß SEQ ID No. 130 ist.

8. Antikörper nach einem der Ansprüche 1 bis 7, wobei die gegen CD3 gerichtete Bindungseinheit die folgende Struktur V_{L}-(G₄S)₃-V_{H} aufweist, wobei V_{L} die variable Region der leichten Kette und V_{H} die variable Region der schweren Kette ist und wobei (G₄S)₃ ein Linkerpeptid gemäß SEQ ID No. 131 ist.

9. Antikörper nach einem der Ansprüche 1 bis 8 zur Verwendung als Arzneimittel, insbesondere zur Behandlung von Tumorerkrankungen, bevorzugt Prostatakrebs, oder als Diagnostikum.

10. Nukleinsäure, dessen Nukleotidsequenz für einen rekombinanten Antikörper nach einem der Ansprüche 1 bis 8 kodiert.

11. Vektor enthaltend eine Nukleotidsequenz nach Anspruch 10.

12. Wirtszelle oder nicht-menschlicher Wirtsorganismus enthaltend eine Nukleotidsequenz nach Anspruch 10 oder einen Vektor nach Anspruch 11.

13. Pharmazeutische Zusammensetzung enthaltend einen rekombinanten Antikörper nach einem der Ansprüche 1 bis 8 und ein pharmazeutisch annehmbares Verdünnungsmittel oder Träger, bevorzugt in einer für die intravenöse Verabreichung geeigneten Form.

## Claims

1. Antibody comprising at least two different binding units, wherein
i. at least one of the binding units binds to prostate-specific stem cell antigen (PSCA), wherein
- a variable region of the light chain comprises the amino acid sequences SEQ ID No. 1, SEQ ID No. 2 and SEQ ID No. 3, and
- a variable region of the heavy chain comprises the amino acid sequences: SEQ ID No. 4, SEQ ID No. 5 and SEQ ID No. 6;
ii. at least one other of the binding units is directed against CD3.

2. Antibody according to claim 1, wherein the binding unit directed against CD3 contains:
- a variable region of the heavy chain containing the amino acid sequences SEQ ID No. 66, SEQ ID No. 67 and SEQ ID No. 68, and
- a variable region of the light chain containing the amino acid sequences SEQ ID No. 58, SEQ ID No. 59 and SEQ ID No. 60.

3. Antibody according to claim 1 or 2, whose variable regions are humanized.

4. Antibody according to one of the claims 1 to 3, wherein the binding unit directed against PSCA comprises
(i) a variable region of the heavy chain according to SEQ ID No. 26 or an amino acid sequence with at least 80% sequence identity to SEQ ID No. 26, and
(ii) a variable region of the light chain according to SEQ ID No. 24 or an amino acid sequence with at least 80% sequence identity to SEQ ID No. 24.

5. Antibody according to one of the claims 1 to 4, wherein the binding unit directed against CD3 comprises a variable region of the light chain according to SEQ ID No. 57 and a variable region of the heavy chain according to SEQ ID No. 65.

6. Antibody according to one of the claims 1 to 5 in the form of a diabody, triabody or tetrabody.

7. Antibody according to one of the claims 1 to 6, wherein the binding unit directed against PSCA is an scFv fragment having the following structure V_{L}-G₄SG₄SGASAAG₄SG₄S-V_{H}, wherein V_{L} is the variable region of the light chain and V_{H} is the variable region of the heavy chain and where G₄SG₄SGASAAG₄SG₄S is a linker peptide according to SEQ ID No. 130.

8. Antibody according to one of the claims 1 to 7, wherein the binding unit directed against CD3 has the following structure V_{L}-(G₄S)₃-V_{H}, wherein V_{L} is the variable region of the light chain and V_{H} is the variable region of the heavy chain and where (G₄S)₃ is a linker peptide according to SEQ ID No. 131.

9. Antibody according to one of the claims 1 to 8 for use as medicament, in particular for the treatment of tumor diseases, preferably prostate cancer, or as a diagnostic agent.

10. Nucleic acid whose nucleotide sequence encodes for a recombinant antibody according to one of the claims 1 to 8.

11. Vector containing a nucleotide sequence according to claim 10.

12. Host cell or non-human host organism containing a nucleotide sequence according to claim 10 or a vector according to claim 11.

13. Pharmaceutical composition containing a recombinant antibody according to one of the claims 1 to 8 and a pharmaceutically acceptable thinner or carrier, preferably in a form suitable for intravenous administration.

## Revendications

1. Anticorps comprenant au moins deux éléments de liaison différents, dans lequel
i. au moins un des éléments de liaison se lie à l'antigène de cellule-souche spécifique de la prostate (PSCA) dans lequel
- une région variable de la chaîne légère comprennent les séquences d'acides aminés SEQ ID N° 1, SEQ ID N° 2 et SEQ ID N° 3 et
- une région variable de la chaîne lourde comprennent les séquences d'acides aminés SEQ ID N° 4, SEQ ID N° 5 et SEQ ID N° 6.
ii. au moins un autre des éléments de liaison est dirigée contre le CD3.

2. Anticorps selon la revendication 1, dont l'élément de liaison dirigée contre le CD3 comprent :
- une région variable de la chaîne légère comprenant les séquences d'acides aminés SEQ ID N° 66, SEQ ID N° 67, SEQ ID N° 68 et
- une région variable de la chaîne lourde comprenant les séquences d'acides aminés SEQ ID N° 58, SEQ ID N° 59, SEQ ID N° 60.

3. Anticorps selon la revendication 1 ou 2 régions dont les régions variables sont humanisées.

4. Anticorps selon l'une des revendications 1 à 3, dont l'élément de liaison dirigée contre le PSCA comprent :
(i) une région variable de la chaîne lourde selon SEQ ID N° 26 ou une séquence d'acides aminés présentant une identité de séquence d'au moins 80 % avec SEQ ID N° 26, et
(ii) une région variable de la chaîne légère selon SEQ ID N° 24 ou une séquence d'acides aminés présentant une identité de séquence d'au moins 80 % avec SEQ ID N° 24.

5. Anticorps selon l'une des revendications 1 à 4, dont l'élément de liaison dirigée contre le CD3 comprent une région variable de la chaîne légère selon SEQ ID N° 57 et une région variable de la chaîne lourde selon SEQ ID N° 65.

6. Anticorps selon l'une des revendications 1 à 5, sous forme d'un diabody, d'un triabody ou d'un tetrabody.

7. Anticorps selon l'une des revendications 1 à 6, dans lequel l'élément de liaison dirigée contre la PSCA est un fragment scFv ayant la structure suivante
V_{L}-G₄SG₄SGASAAG₄SG₄S-V_{H},
dans laquelle V_{L} est la région variable de la chaîne légère et V_{H} est la région variable de la chaîne lourde et dans laquelle G₄SG₄SGASAAG₄SG₄S est un peptide de liaison selon SEQ ID No. 130.

8. Anticorps selon l'une des revendications 1 à 7, dans lequel l'élément de liaison dirigée contre CD3 a la structure suivante V_{L}-(G₄S)₃-V_{H}, dans laquelle V_{L} est la région variable de la chaîne légère et V_{H} est la région variable de la chaîne lourde et dans laquelle (G₄S)₃ est un peptide de liaison selon SEQ ID No. 131.

9. Anticorps selon l'une des revendications 1 à 8, pour une utilisation en tant que médicament, en particulier pour le traitement des tumeurs, de préférence le cancer de la prostate, ou en tant que diagnostic.

10. Acide nucléique, dont la séquence de nucléotides code pour un anticorps recombinant selon l'une des revendications 1 à 8.

11. Vecteur contenant une séquence de nucléotides selon la revendication 10.

12. Cellule hôte ou organisme hôte non humain contenant une séquence de nucléotides selon la revendication 10 ou un vecteur selon la revendication 11.

13. Composition pharmaceutique contenant un anticorps recombinant selon l'une des revendications 1 à 8 et un diluant ou un support pharmaceutiquement acceptable, de préférence sous une forme adaptée à l'administration intraveineuse.
